(19) Europäisches Patentamt
European Patent Office
Office européen des brevets

(11) **EP 2 858 716 B1**

(12) **EUROPEAN PATENT SPECIFICATION**

(45) Date of publication and mention
of the grant of the patent:
**12.10.2022 Bulletin 2022/41**

(21) Application number: **13729003.7**

(22) Date of filing: **06.06.2013**

(51) International Patent Classification (IPC):
**A61N 1/37** (2006.01)   **A61N 1/36** (2006.01)
**G01R 33/28** (2006.01)

(52) Cooperative Patent Classification (CPC):
**A61N 1/36142; A61N 1/3718; G01R 33/288**

(86) International application number:
**PCT/EP2013/061715**

(87) International publication number:
**WO 2013/186118 (19.12.2013 Gazette 2013/51)**

(54) **SYNCHRONIZATION OF THE OPERATION OF AN IMPLANTABLE DEEP BRAIN STIMULATION SYSTEM WITH THE APPLICATION OF RF FIELDS IN AN MRI SYSTEM**

SYNCHRONISATION DES BETRIEBS EINES IMPLANTIERBAREN TIEFENHIRNSTIMULATIONSSYSTEMS MIT DER ANWENDUNG VON RF-FELDERN IN EINEM SYSTEM DER BILDGEBENDEN MAGNETISCHEN RESONANZ

SYNCHRONISATION DE L'OPERATION D'UN SYSTÈME DE STIMULATION CÉRÉBRALE PROFONDE IMPLANTABLE AVEC L'APPLICATION DE CHAMPS DE RADIOFRÉQUENCE DANS UN SYSTÈME D'IRM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2012 EP 12171506**
**11.06.2012 US 201261657997 P**

(43) Date of publication of application:
**15.04.2015 Bulletin 2015/16**

(73) Proprietor: **Medtronic Bakken Research Center B.V.**
**6229 GW Maastricht (NL)**

(72) Inventors:
 • **TOL, Jeroen Jacob Arnold**
 **NL-5645 JG Eindhoven (NL)**
 • **BUDZELAAR, Franciscus Paulus Maria**
 **NL-5632 PL Eindhoven (NL)**

(74) Representative: **Dehns**
**St. Bride's House**
**10 Salisbury Square**
**London EC4Y 8JD (GB)**

(56) References cited:
**WO-A1-2011/063848**    **US-A1- 2004 088 012**
**US-A1- 2004 263 172**    **US-A1- 2005 070 787**
**US-A1- 2005 070 975**    **US-A1- 2007 238 975**
**US-A1- 2007 255 332**    **US-A1- 2009 256 574**
**US-A1- 2011 156 706**    **US-A1- 2011 196 449**
**US-A1- 2012 109 260**

• **J.A. Nyenhuis ET AL: "MRI and implanted medical devices: basic interactions with an emphasis on heating", IEEE TRANSACTIONS ON DEVICE AND MATERIALS RELIABILITY., vol. 5, no. 3, 1 September 2005 (2005-09-01), pages 467-480, XP055498651, US ISSN: 1530-4388, DOI: 10.1109/TDMR.2005.859033**

**Description**

The present invention relates to an implantable deep brain stimulation system.

More and more implants require that the patient carrying such a device might undergo an MRI scan during the lifetime of the implant. This not only implies that the device needs to be MRI safe but also that the device survives an MRI scan. Moreover, if the implant cannot be switched off (completely) during an MRI scan for therapeutic reasons or simply because it cannot to be turned on afterwards if one does so, its electronics needs to be designed in such a way that it does not interfere with the picture making process of an MRI machine.

Almost all active electronic devices such as implantables generate high frequency signals.

In particular the digital parts of an implantable, such as microprocessors or digital communication lines, generate square waves that contain harmonic components that are many times the applied clock frequency. MRI scanners can be viewed as extremely sensitive radio receivers for signals around their RF operating frequency, which is approximately 64 MHz for a 1.5 T scanner and 128 MHz for a 3 T scanner. The emitted harmonics which fall in those frequency bands may easily interfere with the imaging process of an MRI machine leading to unusable images.

US 7,660,620 B2 discloses timing techniques for magnetic resonance imaging.

Timing information is received from an implantable medical device and there are means for synchronizing application of electromagnetic radiation with the received timing information and means for imaging cardiac tissue during application of the cardiac stimulation by the implantable medical device based upon the timing information.

US 2010/0106006 A1 discloses a device comprising a telemetry unit to receive information from an implantable medical device, a control unit that defines timing of electromagnetic radiation bursts based on information received from the implantable medical device, and an electromagnetic radiation source that applies electromagnetic radiation bursts to a patient within which the implantable medical device is implanted based on the defined timing. It is possible that information regarding the timing of the electromagnetic radiation bursts is sent to the implantable medical device to control the operation of the implantable medical device.

US 2005/070975 discloses a deep brain stimulator configured to switch to a blanking period upon detection of an RF burst emitted by an MRI scanner.

It is therefore an object of the present invention to improve an electronic system of an implantable deep brain stimulation system such that the electronics of the system are configured such that it will not interfere with the MRI imaging process without switching off the electronics of the system.

The above object is solved by an implantable deep brain stimulation system according to claim 1. Accordingly, an implantable deep brain stimulation system is provided, which is adapted for compatibility with an MRI machine, to prevent an interference with the picture making process of the MRI machine, wherein the MRI machine is configured to operate in a magnetic resonance imaging scanning mode with a radio frequency, RF, cycle time T, wherein radio frequency bursts are emitted for a first portion of cycle time T, and wherein, after the first portion, the MRI machine is in a listening mode,

wherein the deep brain stimulation system comprises an electronic system,
wherein the electronic system comprises a controller configured to switch the electronic system from operation in a normal operating mode to operation in a magnetic resonance imaging mode;
whereby the electronic system further comprises a first circuit for performing functions of the deep brain stimulation system, and a second circuit configured such that this second circuit is switched off in the magnetic resonance imaging scanning mode;
the electronic system further comprising:

an RF burst detector configured to detect, in use, radio frequency bursts emitted by the MRI machine and to provide a synchronization signal; and wherein in the magnetic resonance imaging scanning mode, in response to the synchronization signal, the first circuit is active only during the RF bursts of the MRI machine detected by the RF burst detector, and not during the listening mode portion of the cycle time T.

Such a deep brain stimulation system may be an implantable neurostimulation device, which can be used to treat acute or chronic neurological conditions. Deep brain stimulation (DBS), the mild electrical stimulation of sub-cortical structures, belongs to the category of implantable devices, and has been shown to be therapeutically effective for Parkinson's disease, Dystonia, and Tremor. New applications of DBS in the domain of psychiatric disorders (obsessive compulsive disorder, depression) are being researched and show promising results.

The magnetic resonance imaging apparatus, whose radio frequency bursts may be tracked, may be a magnetic resonance imaging apparatus, which is directly and/or indirectly influencing the electronic system.

According to the invention the advantage may be achieved that the electronics of the deep brain stimulation system are configured such that it will not interfere with the MRI imaging making process without switching off the electronics of the system. J

Consequently, it is not necessary to completely switch off the system.

The deep brain stimulation system comprises at least one magnetic resonance imaging radio frequency burst detection circuit, which is configured such that radio frequency bursts of a magnetic resonance imaging apparatus are detectable by this magnetic resonance imaging radio frequency burst detection circuit.

The magnetic resonance imaging radio frequency burst detection circuit comprises a magnetic resonance imaging (MRI) radio frequency (RF) burst detector.

The MRI RF burst detector may be configured such that it outputs a signal/flag on its burst line to the at least one first circuit when an RF burst is detected. This circuit can then be turned on as long as this flag is present. Of course, this circuit can also ignore this signal if it is not necessary for the circuit to be turned on.

Moreover, the electronic system is configured such that the electronic system may operate in a normal operating mode and a magnetic resonance imaging scanning mode, whereby the electronic system further comprises at least one switching means configured such that the switching means may switch the electronic system between the normal operating mode of the electronic system and the magnetic resonance imaging scanning mode of the electronic system.

The normal operating mode of the electronic system can be an operating mode, wherein all functions of the electronic system and also all functions of the deep brain stimulation system are available. The magnetic resonance imaging scanning mode of the electronic system may be an operating mode of the electronic system and the deep brain stimulation system, whereby only the necessary and essential functions of the deep brain stimulation system are available.

The electronic system further comprises at least one second circuit configured such that this second circuit is switched off completely in the magnetic resonance imaging scanning mode.

Additionally, it is possible that the electronic system further comprises at least one detection means, whereby the detection means is configured such that the presence of a magnetic resonance imaging scanner can be detected automatically. Thereby, the advantage is achieved that no manual switching operation has to be conducted to switch the electronic system between the normal operating mode of the electronic system and the magnetic resonance imaging scanning mode of the electronic system.

Furthermore, the switching means may be configured such that depending on a detection signal of the detection means the electronic system can be switched between normal operating mode and magnetic resonance imaging scanning mode.

In a preferred embodiment it is possible that the detection means is configured such that a magnetic resonance imaging detection signal is generated, when the static magnetic field of the magnetic resonance imaging machine is detected. Since any magnetic resonance imaging machine has a normally extremely strong static magnetic field, the presence of this field can be detected by the detection means and thus the electronic system is able to detect a magnetic resonance imaging machine.

Additionally, it is possible that the detection means advantageously comprises a Hall sensor means. A sensor means, which may be e.g. embodied as a simple hall sensor, allows an accurate and secure detection of a normally extremely strong static magnetic field of the magnetic resonance imaging machine. In particular, the static magnetic field of the magnetic resonance imaging machine may have a static magnetic field of about 1.0 T to 3.0 T. But it is also possible that the static magnetic field is above 3.0 T.

It is possible that the detection means may be configured such that a detection signal is generated when the first radio frequency burst of the MRI apparatus and/or the gradient magnetic field as flag to switch to magnetic resonance imaging mode is detected.

Moreover, the switching means may comprise a switching back means, whereby the switching back means comprises at least one first time out means and/or at least one second time out means.

The first time out means may be configured such that the first time out means is capable to provide the switching means with at least one signal to switch the electronic system to the magnetic resonance imaging scanning mode for a time out phase and to switch the electronic system back to normal operating mode after the time out phase.

The second time out means may be configured such that the second time out means is triggered by a detected radio frequency burst and/or gradient magnetic field by the magnetic resonance imaging radio frequency burst detection circuit and that the second time out means thereafter provides the switching means with at least one signal to switch to magnetic resonance imaging scanning mode for a time out phase and switch back to normal mode after a time out phase.

It is also possible that the switching back means is configured such that it can be manually activated such that a switch back to normal mode can be conducted.

Moreover, the electronic system may further comprise at least one first radio frequency burst time out timer means, which is configured such that it is directly and/or indirectly assured by the radio frequency burst time out timer means that circuit activity of the first circuit is switched off when or before the radio frequency burst ends.

The at least one first radio frequency burst time out timer means may be embodied as or comprise at least one radio frequency (RF) burst time out timer.

The RF burst time out timer may be e.g. notified by the magnetic resonance imaging radio frequency burst detection circuit, via a signal on its output burst line, that an RF burst is being detected, and therefore, can measure the duration of a detected RF burst.

The time out timer can be set to this measured duration, or any smaller fraction of it, before the subsequent RF burst is detected.

Thus when time out timer is triggered by the magnetic resonance imaging radio frequency burst detection circuit when the subsequent RF pulse is detected, it can be assured that the first circuit is completely switched off when or before this subsequent RF burst ends.

For instance, one can either keep measuring the duration of every next RF burst as timer input for the subsequent RF pulse, measure once and use this value for all next detected RF bursts (i.e. for the whole duration that electronic system is in MRI mode), or finally, one could also opt to pre-program the time out phase duration and use this every time an RF burst is detected by the magnetic resonance imaging radio frequency burst detection circuit.

For the first detected RF burst, the at least one first RF burst time out timer may simply pass the signal on the burst line of the at least one magnetic resonance imaging radio frequency burst detection circuit to the first circuit directly as if the timer were not present.

Additionally, it is possible that the electronic system further comprises at least one second radio frequency burst time out timer means, which is configured such that the presence of an externally applied signal is directly and/or indirectly detected by the second frequency burst time out timer means and that by this the second frequency burst time out timer means is controlled and that circuit activity of the first circuit is switched off when the time out signal of the second frequency burst time out timer means is generated, whereby the time out signal is generated before the radio frequency burst has decayed away.

The second radio frequency burst time out timer means may be e.g. equipped with an RF antenna. In this way, the second radio frequency burst time out timer means might be notified, or once in a while notified, if this second time out timer means has an internal clock for synchronization, when the MRI machine is transmitting an RF pulse.

The second time out timer may use the externally applied signal in the same way the first time out timer applies the detection signal from the magnetic resonance imaging radio frequency burst detection circuit.

The externally applied signal may also be used to control the circuit activity of the first circuit directly e.g. the externally applied signal may be used to switch the first circuit on and off directly instead of via the second time out timer.

By the features of the inventive deep brain stimulation system, e.g. the advantage can be achieved that the deep brain stimulation system must not be completely switched off during a magnetic resonance imaging process. Additionally, the magnetic resonance imaging process is not affected by the deep brain stimulation system e.g. due to interferences and thus magnetic resonance imaging can be conducted with high quality.

Further details and advantages of the present invention shall be described hereinafter with respect to the drawings:

Fig. 1: a schematical drawing of a typical radio frequency (RF) waveform;

Fig. 2a: a schematical drawing of a first embodiment of the electronic system according to the present invention;

Fig. 2b: a schematical drawing of a modification of the first embodiment of the electronic system that does not form part of the claimed invention;

Fig. 3: a schematical drawing of a second embodiment of the electronic system according to the present invention;

Fig. 4: a schematical drawing of a third embodiment of the electronic system according to the present invention;

Fig. 5: a schematical drawing of a fourth embodiment of the electronic system according to the present invention;

Fig. 6: a schematical drawing of a fifth embodiment of the electronic system according to the present invention;

Fig. 7: a schematical drawing of a sixth embodiment of the electronic system according to the present invention;

Fig. 8: a schematical drawing of a first example of the electronic system that does not form part of the claimed invention;

Fig. 9: a schematical drawing of a second example of the electronic system that does not form part of the claimed invention;

Fig. 10: a schematical drawing of a third example of the electronic system that does not form part of the claimed invention;

Fig. 11: a schematical drawing of a fourth example of the electronic system that does not form part of the claimed invention;

Fig. 12: a schematical drawing of a typical worst-case gradient waveform for a 1.0 T MRI system;

Fig. 13: a schematical drawing of a neurostimulation system for deep brain stimulation (DBS);

Fig. 14: a further schematical drawing of a probe neurostimulation system for deep brain stimulation (DBS) and its components; and

Fig. 15: a schematical drawing of a probe system according to the present invention.

Magnetic resonance imaging scanners perform their imaging in a periodic fashion. A strong static magnetic field aligns the spins in the nuclei of the investigated tissue of the scanned person. A radio frequency (RF) field is superimposed to flip the spins that were aligned by the static magnetic field. This RF field is composed of a short, very powerful burst absorbed by the tissue of the scanned person. For the excitation of hydrogen nuclei (and thus e.g. also water), the frequency is 42.57 MHz/T or approximately 64 MHz for a 1.5 T system, approximately 128 MHz for a 3.0 T system. After the RF burst, a magnetic resonance imaging (MRI) machine switches to "listening" mode to detect the radio waves emitted by the tissue as the (hydrogen) nuclei revert to their original spin state. The frequency of these very weak radio waves is the same as the frequency of the initially superimposed RF field (i.e. 64 MHZ for a 1.5 T scanner). This is the reason why an MRI machine is so extremely sensitive for any emitted electromagnetic radiation, for example by an implant, in the RF band of the scanner.

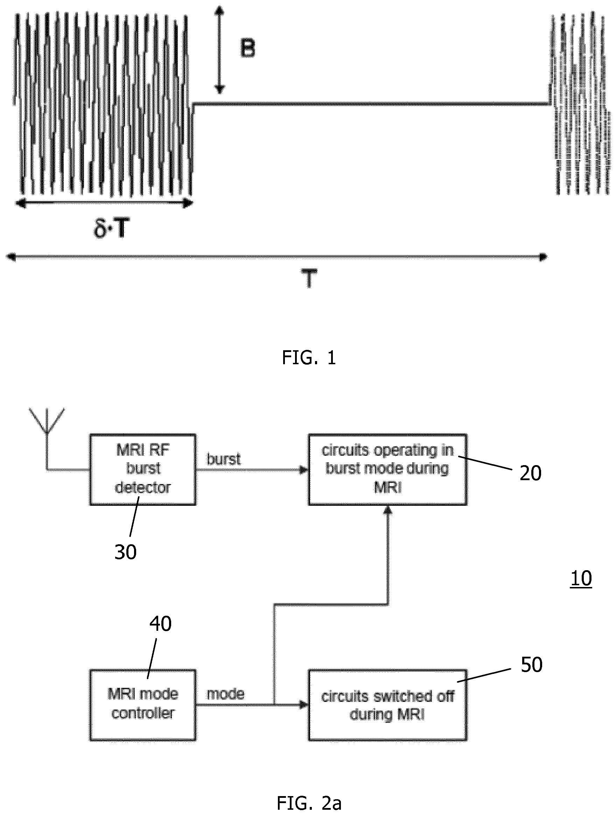

Figure 1 depicts an RF burst. The typical RF waveform is showing its high frequency sinusoidal burst character with a duration $\delta T$, the period T and the magnetic filed strength amplitude B.

The total RF cycle time is T, which is typically in the order of 10ms. The RF burst itself typically takes less than 5 % (i.e. < 0.05 typically) of the total time T. In the remaining time, the MRI scanner is in listening mode.

Figure 2a shows schematically a first embodiment of the electronic system 10 according to the present invention.

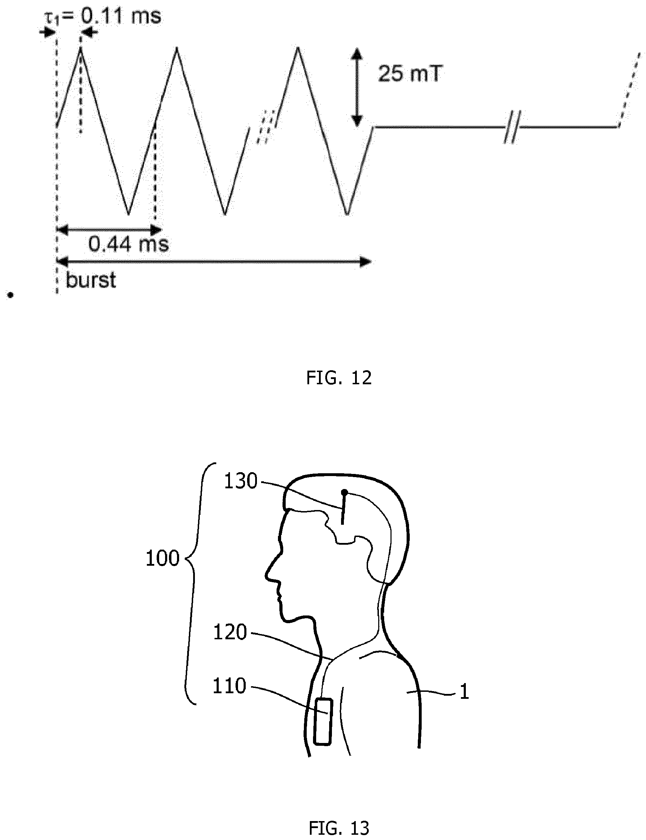

The electronic system 10 is an electronic system 10 for a deep brain stimulation system 100 (see Figures 13 to 15). The electronic system 10 is configured such that the electronic system 10 may operate in a normal operating mode and a magnetic resonance imaging scanning mode, whereby the electronic system 10 further comprises at least one first circuit 20 configured such that the at least one first circuit 20 operates in a duty-cycle mode, during a magnetic resonance imaging scan.

Moreover, the electronic system 10 has at least one magnetic resonance imaging radio frequency (MRI RF) burst detection circuit 30 and at least one switching means 40 configured such that the switching means may switch the electronic system 10 between the normal operating mode of the electronic system 10 and the magnetic resonance imaging scanning mode of the electronic system 10. The switching means 40 is embodied as a MRI mode controller 40.

The MRI mode controller 40 puts the electronic system 10 in its "MRI" mode which implies that some parts are switched off completely, while other parts are turned on periodically every time the MRI machine sends out an RF burst.

The switching means 40, respectively the MRI mode controller 40 may be configured such that the switching means 40 is connectable to an external system, whereby the external system forces the switching means 40 to switch between the normal operating mode and the magnetic resonance imaging scanning mode, whereby the switching means 40 is preferably equipped with radio frequency means and/or inductive communication means.

The electronic system 10 further comprises at least one second circuit 50 configured such that this second circuit 50 is switched off completely during a magnetic resonance imaging scan.

The MRI RF burst detection circuit 30 is embodied as MRI RF burst detector 30 and by means of the MRI RF burst detector 30 the occurrence of an RF burst can easily be detected. It is assumed that the activity of circuit 20 during an RF burst has no effect on the quality of the MRI scan. Consequently, one can operate electronic circuits for some (essential) functions during MRI imaging if this circuit activity is synchronized with the emitted RF bursts of an MRI machine.

Therefore, it is possible with electronic system 10 shown in Figure 2a that some electronics 50 is switched off completely, and the remaining electronic circuits 20 operate in sync with the RF bursts of the MRI scanner so that the circuits 20 are only active at the moment the MRI scanner is not sensitive for the potential interference generated by the activity of the circuits 20. As synchronization signal for the circuits 20, the output signal burst of the RF detector 30 is applied.

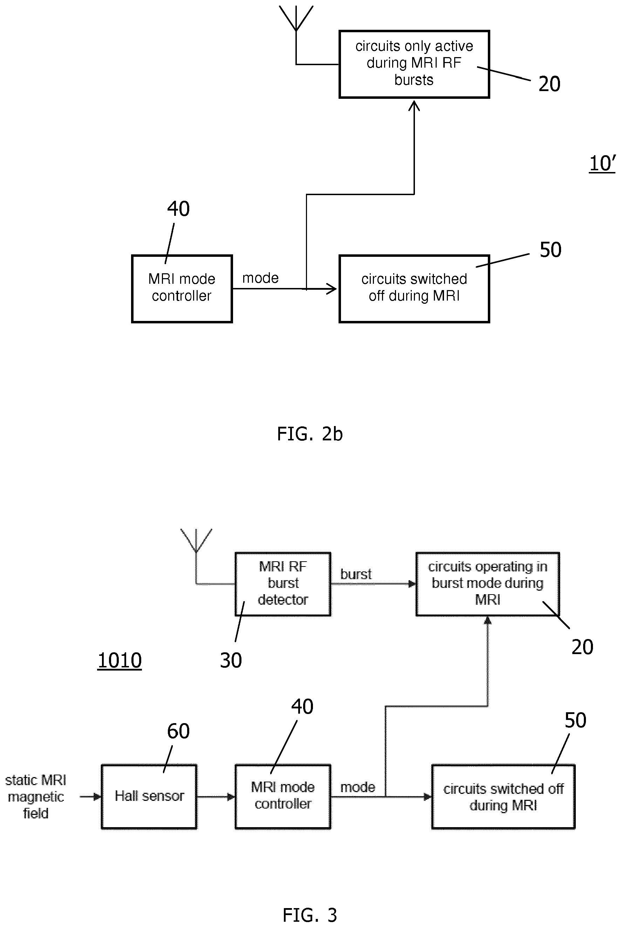

Figure 2b shows a modification of the first embodiment of the electronic system 10' that does not form part of the claimed invention. All components of the electronic system 10' are the same as of the electronic system 10 as shown in Figure 2a. As shown in Figure 2b, it is also possible, to provide the at least one circuit 20 with an external signal detecting means, e.g. an RF receiver, which is included into the circuit 20. So the circuit 20 may be directly controlled via an externally applied signal. For instance, the externally applied signal may be used to switch the first circuit on and off directly.

Figure 3 shows schematically a second embodiment of the electronic system 1010 according to the present invention, which comprises all elements of the first embodiment of the electronic system 10 shown in Figure 2a and which is equipped with further advantageous optional features.

The electronic system 1010 also comprises the first circuit 20, MRI RF burst detector 30, a MRI mode controller 40 as switching means 40 and second circuit 50.

Furthermore, the electronic system 1010 further comprises at least one detection means 60, whereby the detection means 60 is configured such that the presence of a magnetic resonance imaging scanner can be detected automatically. The detection means of the embodiment shown in Figure 3 is a Hall sensor 60, which generates a magnetic resonance imaging detection signal, when the static magnetic field of the magnetic resonance imaging machine is detected.

The switching means 40 is configured such that depending on a detection signal of the Hall sensor 60 the electronic system 1010 may be switched between normal operating mode and magnetic resonance imaging scanning mode automatically. The sensitivity of the Hall sensor 60 is configured such that the MRI mode controller 40 is not falsely triggered by other external magnetic fields than the static MRI field under normal operating conditions (i.e. daily life).

In both embodiments shown in Figure 2a and 3 it is possible that the switching means 40 comprises a switching back means, whereby the switching back means comprises at least one first time out means, whereby the first time out means is configured such that the first time out means is capable to provide the switching means 40 with at least one signal to switch the electronic system 10 or 1010 to the magnetic resonance imaging scanning mode for a time out phase and to switch the electronic system 10 or 1010 back to normal operating mode after the time out phase automatically.

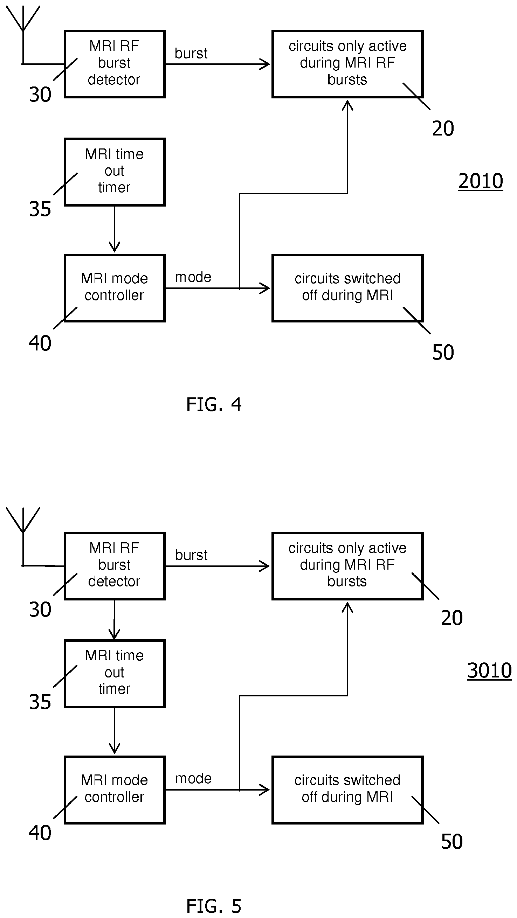

Figure 4 shows schematically a third embodiment of the electronic system 2010 according to the present invention, which comprises all elements of the first embodiment of the electronic system 10 shown in Figure 2a and which is equipped with further advantageous optional features.

The electronic system 2010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is embodied as a stand alone MRI time out timer and provides the MRI mode controller 40 with at least one signal to switch to magnetic resonance imaging scanning mode for a time out phase and switch back to normal mode after a time out phase automatically.

Figure 5 shows schematically a fourth embodiment of the electronic system 3010 according to the present invention, which comprises all elements of the first embodiment of the electronic system 10 shown in Figure 2a and which is equipped with further advantageous optional features.

The electronic system 10 also comprises the first circuit 20, MRI RF burst detector 30, a MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

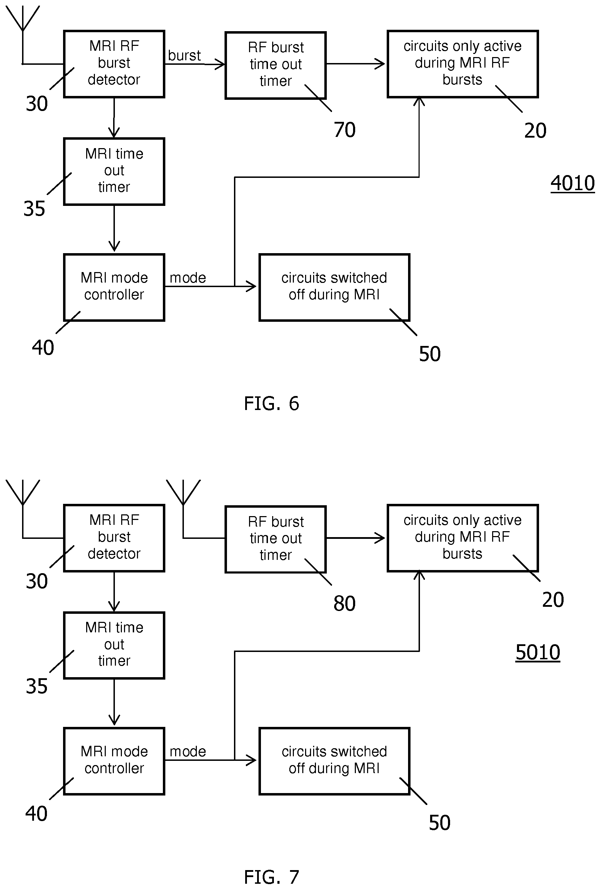

Figure 6 shows schematically a fifth embodiment of the electronic system 4010 according to the present invention, which comprises all elements of the first embodiment of the electronic system 10 shown in Figure 2a and which is equipped with further advantageous optional features.

The electronic system 4010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

Moreover, the electronic system 4010 further comprises at least one first radio frequency burst time out timer means 70, which is configured such that it is directly and/or indirectly assured by the radio frequency burst field time out timer means 70 that circuit activity of the first circuit 20 is switched off when or before the radio frequency burst ends.

The at least one first radio frequency burst time out timer means 70 may be embodied as or comprise at least one radio frequency (RF) burst time out timer.

The RF burst time out timer may be e.g. notified by the magnetic resonance imaging radio frequency burst detection circuit 30, via a signal on its output burst line, that an RF burst is being detected, and therefore, can measure the duration of a detected RF burst. The time out timer 70 can be set to this measured duration, or any smaller fraction of it, before the subsequent RF burst is detected. Thus when time out timer 70 is triggered by the magnetic resonance imaging radio frequency burst detection circuit 30 when the subsequent RF pulse is detected, it can be assured that first circuit 20 is completely switched off when or before this subsequent RF burst ends.

For instance, one can either keep measuring the duration of every next RF burst as timer input for the subsequent RF pulse, measure once and use this value for all next detected RF bursts (i.e. for the whole duration that electronic system 4010 is in MRI mode), or finally, one could also opt to pre-program the time out phase duration and use this every time an RF burst is detected by the magnetic resonance imaging radio frequency burst detection circuit 30.

For the first detected RF burst, the at least one first RF burst time out timer may simply pass the signal on the burst line of the at least one magnetic resonance imaging radio frequency burst detection circuit to the first circuit directly as if the timer were not present.

Figure 7 shows schematically a sixth embodiment of the electronic system 5010 according to the present invention, which comprises all elements of the first embodiment of the electronic system 10 shown in Figure 2a and which is equipped with further advantageous optional features.

The electronic system 5010 also comprises the first circuit 20, MRI RF burst detector 30, an MRI mode controller 40 as switching means 40 and second circuit 50. Furthermore, a time out timer 35 is provided, which forms the so-called second time out means of the switching back means.

The time out timer 35 is configured such that it is triggered by a detected radio frequency burst detected by the MRI RF burst detector 30, preferably the first detected radio frequency burst, and that it thereafter provides the MRI mode controller 40 with at least one signal to switch to magnetic resonance imaging scanning mode for a time out phase and switch back to normal mode after a time out phase automatically.

As shown in Figure 7, it is possible that the electronic system 5010 further comprises at least one second radio frequency burst time out timer means 80, which is configured such that the presence of an externally applied signal is directly and/or indirectly detected by the second frequency burst time out timer means 80 and that by this the second frequency burst time out timer means 80 is controlled and that circuit activity of the first circuit 20 is switched off when the time out signal of the second frequency time out timer means 80 is generated. In any case in this embodiment, the time out signal is generated before the radio frequency burst has decayed away.

The second radio frequency burst time out timer means may be e.g. equipped with an RF antenna. In this way, the second radio frequency burst time out timer means 80 might be notified, or once in a while notified, if this second time out timer means 80 has an internal clock for synchronization, when the MRI machine is transmitting_an RF pulse.

The second time out timer may use the externally applied signal in the same way the first time out timer applies the detection signal from the magnetic resonance imaging radio frequency burst detection circuit.

The externally applied signal may also be used to control the circuit activity of the first circuit directly e.g. the externally applied signal may be used to switch the first circuit on and off directly instead of via the second time out timer.

The option to use a time out timer 35 as shown in the embodiment of Figure 4 to 7 can be extended by a means to put the electronics back into normal operating mode manually, immediately after the person has undergone an MRI scan.

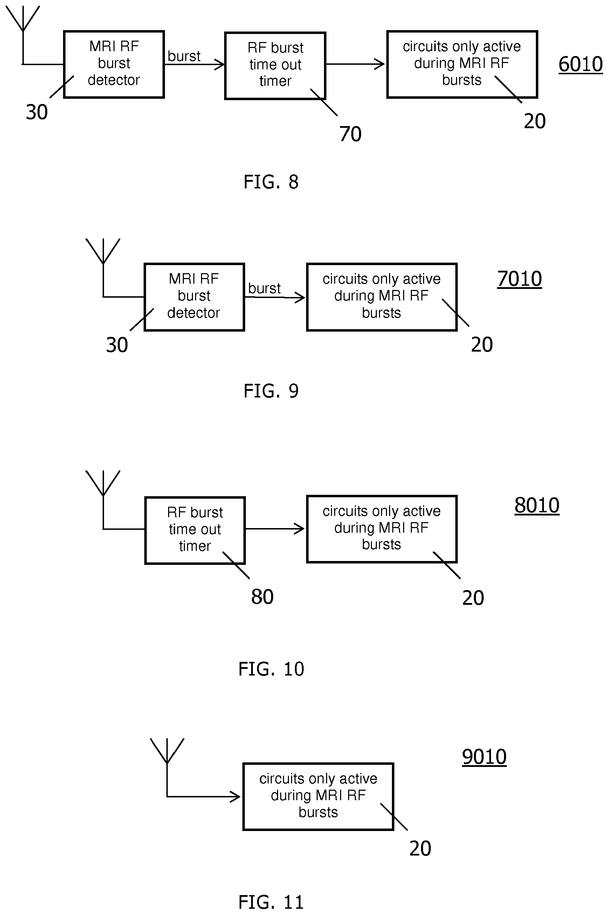

Figures 8 to 11 show single mode systems. These systems are "MRI only mode" systems, and do not form part of the claimed invention.

The first option is shown in Figure 8, which shows the electronic system 6010.

The electronic system 6010 comprises an MRI RF burst detector 30, an RF burst time out timer 70 and at least one first circuit 20. Likewise the embodiment shown in Figure 2a, the electronic circuit 20 operates in sync with the RF bursts of the MRI scanner so that the circuit 20 (or circuits 20) are only active at the moment the MRI scanner is not sensitive for the potential interference generated by the activity of the circuits 20. As synchronization signal for the circuits 20, the output signal burst of the RF detector 30 is applied.

The RF burst time out timer 70 may be an RF burst and/or gradient magnetic field time out timer 70 which is notified by magnetic resonance imaging radio frequency burst detection circuit 30, via a signal on its output burst line, that an RF burst and/or gradient magnetic field is being detected, and therefore, can measure the duration of a detected RF burst and/or gradient magnetic field pulse.

The time out timer can be set to this measured duration, or any smaller fraction of it, before the subsequent RF burst and/or gradient magnetic field is detected. Thus when time out timer 70 is triggered by detection circuit 30 when the subsequent RF pulse and/or gradient magnetic field is detected, it can be assured that the first circuit 20 is completely switched off when or before this subsequent RF burst and/or gradient magnetic field pulse ends.

One can either keep measuring the duration of every next RF burst and/or gradient magnetic field pulse as timer input for the subsequent RF pulse and/or gradient magnetic field pulse, measure once and use this value for all next detected RF bursts and/or gradient magnetic field pulses (i.e. for the whole duration that system 6010 is in MRI mode), or finally, one could also opt to pre-program the time out phase duration and use this every time an RF burst and/or gradient magnetic field is detected by circuit 30.

For the first detected RF burst and/or gradient magnetic field pulse, the at least one first RF burst and/or gradient magnetic field time out timer may simply pass the signal on the burst line of the at least one magnetic resonance imaging radio frequency burst detection circuit to the first circuit directly as if the timer were not present.

The second option is shown in Figure 9, which shows the electronic system 7010. The electronic system 7010 comprises an MRI RF burst detector 30 and at least one first circuit 20. Likewise the embodiment shown in Figure 2a or 8, the electronic circuit 20 operates in sync with the RF bursts of the MRI scanner so that the circuit 20 (or circuits 20) are only active at the moment the MRI scanner is not sensitive for the potential interference generated by the activity of the circuits 20. As synchronization signal for the circuits 20, the output signal burst of the RF detector 30 is applied.

The third option is shown in Figure 10, which shows the electronic system 8010. The electronic system 8010 comprises at least one second radio frequency burst and/or gradient magnetic field time out timer means 80 and at least one first circuit 20. Likewise the embodiment shown in Figure 7, at least one second radio frequency burst and/or gradient magnetic field time out timer means 80, which is configured such that the presence of an externally applied signal is directly and/or indirectly detected by the second frequency burst and/or gradient magnetic field time out timer means 80 and that by this the second frequency burst and/or gradient magnetic field time out timer means 80 is controlled and that circuit activity of the first circuit 20 is switched off when the time out signal of the second frequency burst and/or gradient magnetic field time out timer means 80 is generated. In any case in this 2. example that does not form part of the claimed invention, the time out signal is generated before the radio frequency burst and/or gradient field has decayed away.

The fourth option is shown in Figure 11, which shows the electronic system 9010.

The electronic system 9010 comprises at least one first circuit 20, which is modified. Likewise the embodiment shown in Figure 2b, this modified circuit 20 is equipped with an external signal detecting means, e.g. an RF receiver, which is included into the circuit 20. So the circuit 20 may be directly controlled via an externally applied signal. For instance, the externally applied signal may be used to switch the first circuit on and off directly.

In all embodiments 2. of the invention and examples that do not form part of the claimed invention described above and shown in Figures 2 to 9 it is possible that the MRI RF burst detector 30 can consist of a magnetic antenna (i.e. coil) and an RF detector. The RF frequency $f_{RF}$ of the MRI scanner is related to the field strength $B_{MRI}$ of the static field:

$$f_{RF}(B_{MRI}) = 42.58 * B_{MRI} * \frac{MHz}{T}$$

The maximum field strength $B_{max}$ of the RF field varies spatially and is also related to the type of scanner. In the centre of an MRI scanner, the amplitude can be up to 27 $\mu$T for 1.5 T systems and up to 13 $\mu$T in 3 T MRI machines. Close to the rings of the RF coil, the amplitude of the RF field is the highest and can be up to 45 $\mu$T. For this maximum amplitude $B_{max}$ of

$$B_{max} = 45 \mu T$$

the maximum dB/dt is

$$\frac{dB}{dt}_{max} = 2 * \pi * B_{max} * f_{RF}$$

which for a 1.5 T scanner translates into

$$\frac{dB}{dt}_{max,1.5T} = 1.80588 * 10^4 \frac{kg}{A * s^3}$$

and which for a 3.0 T scanner translates into

$$\frac{dB}{dt}_{max,3.0T} = 3.61176 * 10^4 \frac{kg}{A * s^3}$$

The maximum induced voltage $V_{max}$ in a single turn coil with area A is

$$V_{max} = A * \frac{dB}{dT}_{max}$$

and with only a surface area of 1 cm$^2$ one gets

$$V_{max,1.5T} = 1.8\, V$$

and

$$V_{max,3.0T} = 3.6\, V$$

These signal levels can easily be detected by a simple rectification circuit and level detector, even when they are an order of magnitude smaller (i.e. if the electronics is not located near the "hot spot" RF coils). The detector 30 can be designed without disturbing the picture making process during the "listening" mode of the MRI scanner.

False triggers of the burst detector can be prevented by not only setting the detector threshold level appropriately (optionally depending of the type of MRI machine and/or adaptively) but also by using a tuned coil circuit (i.e. filtering at the RF frequency of the MRI machine at hand).

Gradient fields are applied for spatial encoding of the image. Typically, their frequency range is between 0 Hz and 5 kHz. The waveforms can have very different shapes and are certainly not always sinusoidal. The amplitudes of the gradient fields vary strongly in the patient space inside the MRI scanner. The amplitudes are limited by requirements on nerve stimulation.

As an illustration for the case of a 1.0 T "open" MRI machine, the strongest appearing gradient field is shown in Figure 12. In general, the amplitude of a gradient field is typically 3 orders of magnitude larger ($\mu$T vs. mT) than the RF field strength, while their frequency is about 4 to 5 orders smaller (64 MHz vs. 0-5 kHz). Therefore, the induced voltages, proportional to field strength and frequency, are 1 to 2 orders smaller than for the RF fields.

Theoretically, and not forming part of the claimed invention, one could also use the gradient fields instead of the RF bursts to switch (part of) the electronics on periodically during MRI scanning but the gradient field detector must have a much higher sensitivity due to the much lower induced voltages.

However, more importantly, the gradient field burst may also appear during the listening mode of an MRI scanner, depending on the chosen settings during scanning, which implies that the switched electronics might interfere with the picture making process. Therefore, the gradient fields are not suitable for detection purposes in general.

The whole electronics of the deep brain stimulation (DBS) system 100 may be formed by the electronic system 10 as described above. Such a DBS system 100 may be embodied at least partially as shown in Figures 13 to 15 and as described below.

A possible embodiment of a neurostimulation system 100 for deep brain stimulation (DBS) is shown in Figure 13. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

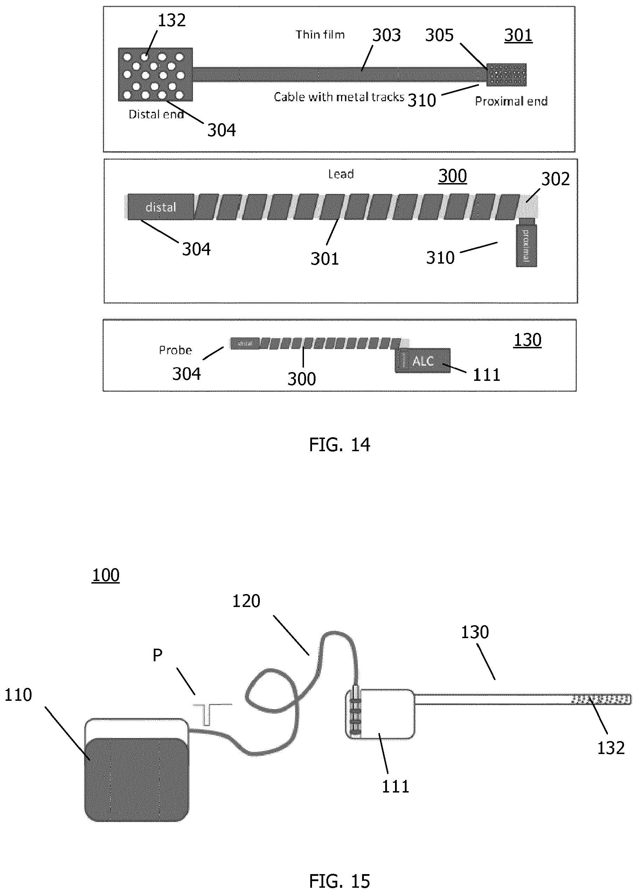

Figure 14 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrodes 132 to a designated proximal contact 305.

Figure 15 shows schematically and in greater detail an embodiment of a system 100 for brain applications, here for neurostimulation and/or neurorecording as a deep brain stimulation system 100 as shown in Figures 13 and 14. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

## Claims

1. An implantable deep brain stimulation system (100) which is adapted for compatibility with an MRI machine, to prevent an interference with the picture making process of the MRI machine, wherein the MRI machine is configured to operate in a magnetic resonance imaging scanning mode with a radio frequency, RF, cycle time T, wherein radio frequency bursts are emitted for a first portion of cycle time T, and wherein, after the first portion, the MRI machine is in a listening mode,

    wherein the deep brain stimulation system (100) comprises an electronic system (10, 1010, 2010, 3010, 4010, 5010), wherein the electronic system comprises a controller (40) configured to switch the electronic system (10, 1010, 2010, 3010, 4010, 5010) from operation in a normal operating mode to operation in a magnetic resonance imaging mode;
    whereby the electronic system (10, 1010, 2010, 3010, 4010, 5010) further comprises a first circuit (20) for performing functions of the deep brain stimulation system (100), and a second circuit (50) configured such that this second circuit (50) is switched off in the magnetic resonance imaging scanning mode;
    the electronic system (10, 1010, 2010, 3010, 4010, 5010) further comprising:

        an RF burst detector (30) configured to detect, in use, radio frequency bursts emitted by the MRI machine and to provide a synchronization signal; and
        wherein in the magnetic resonance imaging scanning mode, in response to the synchronization signal, the first circuit is active only during the RF bursts of the MRI machine detected by the RF burst detector (30), and not during the listening mode portion of the cycle time T.

## Patentansprüche

1. Implantierbares System (100) für eine tiefe Hirnstimulation, das für eine Kompatibilität mit einer MRT-Anlage angepasst ist, um eine Interferenz mit dem Bildaufnahmeverfahren der MRT-Anlage zu verhindern, wobei die MRT-Anlage konfiguriert ist, um in einem Magnetresonanzbildgebungsabtastmodus mit einer Hochfrequenz(HF)-Zykluszeit T betrieben zu werden, wobei Hochfrequenzimpulsfolgen für einen ersten Anteil von Zykluszeit T emittiert werden und wobei, nach dem ersten Anteil, die MRT-Anlage in einem Hörmodus ist,

    wobei das System (100) für die tiefe Hirnstimulation ein elektronisches System (10, 1010, 2010, 3010, 4010, 5010) umfasst, wobei das elektronische System eine Steuervorrichtung (40) umfasst, die konfiguriert ist, um das elektronische System (10, 1010, 2010, 3010, 4010, 5010) von einem Betrieb in einem normalen Betriebsmodus zu einem Betrieb in einem Magnetresonanzbildgebungsmodus zu stellen; wodurch das elektronische System (10, 1010, 2010, 3010, 4010, 5010) ferner eine erste Schaltung (20) zum Durchführen von Funktionen des Systems (100) für die tiefe Hirnstimulation und eine zweite Schaltung (50) umfasst, die derart konfiguriert ist, dass diese zweite Schaltung (50) in dem Magnetresonanzbildgebungsabtastmodus abgestellt ist; wobei das elektronische System (10,1010, 2010, 3010, 4010, 5010) ferner umfasst:

einen HF-Impulsfolgendetektor (30), der konfiguriert ist, um, in Verwendung, Hochfrequenzimpulsfolgen zu erfassen, die durch die MRT-Anlage emittiert werden, und um ein Synchronisationssignal bereitzustellen; und

wobei in dem Magnetresonanzbildgebungsabtastmodus die erste Schaltung als Reaktion auf das Synchronisationssignal nur während der HF-Impulsfolgen der MRT-Anlage, die durch den HF-Impulsfolgendetektor (30) erfasst werden, und nicht während des Hörmodusanteils der Zykluszeit T aktiv ist.

## Revendications

1. Système de stimulation cérébrale profonde (100) implantable qui est adapté pour être compatible avec une machine d'IRM, pour empêcher une interférence avec le processus de création d'image de la machine d'IRM, dans lequel la machine d'IRM est configurée pour fonctionner dans un mode de balayage d'imagerie par résonance magnétique avec un temps de cycle T de radiofréquence, RF, dans lequel des salves de radiofréquence sont émises pendant une première partie du temps de cycle T, et dans lequel, après la première partie, l'appareil d'IRM est dans un mode d'écoute,

dans lequel le système de stimulation cérébrale profonde (100) comprend un système électronique (10, 1010, 2010, 3010, 4010, 5010), le système électronique comprenant un dispositif de commande (40) configuré pour faire passer le système électronique (10, 1010, 2010, 3010, 4010, 5010) d'un fonctionnement dans un mode de fonctionnement normal à un fonctionnement dans un mode d'imagerie par résonance magnétique ; le système électronique (10, 1010, 2010, 3010, 4010, 5010) comprenant en outre un premier circuit (20) pour exécuter des fonctions du système de stimulation cérébrale profonde (100), et un second circuit (50) configuré de telle sorte que ce second circuit (50) est désactivé dans le mode de balayage d'imagerie par résonance magnétique ; le système électronique (10, 1010, 2010, 3010, 4010, 5010) comprenant en outre :

un détecteur de salves RF (30) configuré pour détecter, en cours d'utilisation, des salves de radiofréquence émises par l'appareil d'IRM et pour fournir un signal de synchronisation ; et

dans lequel, dans le mode de balayage d'imagerie par résonance magnétique, en réponse au signal de synchronisation, le premier circuit est actif uniquement pendant les salves RF de l'appareil d'IRM détectées par le détecteur de salves RF (30), et non pendant la partie mode d'écoute du temps de cycle T.

FIG. 1

FIG. 2a

circuits only active
during MRI RF
bursts

20

10′

40

MRI mode
controller

mode

circuits switched
off during MRI

50

FIG. 2b

MRI RF
burst
detector

burst

circuits operating in
burst mode during
MRI

1010

30

20

40

60

static MRI
magnetic
field

Hall sensor

MRI mode
controller

mode

circuits switched off
during MRI

50

FIG. 3

FIG. 4

FIG. 5

FIG. 6

FIG. 7

MRI RF burst detector

burst

RF burst time out timer

circuits only active during MRI RF bursts

6010

30

70

20

**FIG. 8**

MRI RF burst detector

burst

circuits only active during MRI RF bursts

7010

30

20

**FIG. 9**

RF burst time out timer

circuits only active during MRI RF bursts

8010

80

20

**FIG. 10**

circuits only active during MRI RF bursts

9010

20

**FIG. 11**

FIG. 12

FIG. 13

132

Thin film   303   305   301

Distal end

304

Cable with metal tracks 310

Proximal end

Lead   300   302

distal

304   301   310   proximal

Probe   130

304   300   ALC   111

FIG. 14

100

120

P   130

110

111   132

FIG. 15

**EP 2 858 716 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7660620 B2
- US 20100106006 A1
- US 2005070975 A